# EUROPEAN PATENT SPECIFICATION

(11) **EP 1 414 496 B1**
(45) Date of publication and mention of the grant of the patent: **20.10.2010**
(21) Application number: 02766983.7
(22) Date of filing: 07.08.2002
(51) Int. Cl.: A61K 45/06, A61P 3/06, A61K 31/215

(54) **PHARMACEUTICAL COMPOSITION CONTAINING A COMBINAITION OF PPAR-ALPHA, PRAVASTATIN AND POLYGLYCOLIZED GLYCERIDE**
ZUSAMMENSETZUNG ENTHALTEND EINE KOMBINATION AUS EINEM PPAR-ALPHA, PRAVASTATIN UND POLYGLYKOLISIERTEM GLYZERID
COMPOSITION PHARMACEUTIQUE CONTENANT UNE COMBINAISON DE PPAR-ALPHA, PRAVASTATIN ET DE GLYCERIDE POLYGLYCOLISE

(30) Priority: 07.08.2001 WO PCT/BE01/00133; 07.09.2001 WO PCT/BE01/00147
(43) Date of publication of application: 06.05.2004
(73) Proprietor: Galephar M/F, 6900 Marche-en-Famenne (BE)
(72) Inventor: VANDERBIST, Francis, B-1650 Beersel (BE); DEBOECK, Arthur, Puerto Rico 00778 (US); BAUDIER, Philippe, B-1180 Uccle (BE); SERENO, Antonio, B-1820 Melsbroek (BE)
(74) Representative: Vandeberg, Marie-Paule L.G.
(86) International application number: PCT/BE2002/000135
(87) International publication number: WO 2003/013608

(56) References cited:
- EP-A- 0 455 042
- EP-A- 0 475 148
- WO-A-00/37078
- WO-A1-96/21439
- US-B1- 6 171 615
- R.L.B.ELLEN, R.MCPHERSON: "Long-term efficacy and safety of fenofibrate and statin in the teatment of combined hyperlipidemia" AMERICAN JOURNAL OF CARDIOLOGY, vol. 81, no. 4A, 1998, pages 60B-65B, XP000925448
- M.FARNIER, S.DEJAGER: "Effect of combined fluvastatin-fenofibrate therapy compared with fenofibrate monotherapy in severe primary hypercholesterolemia" AMERICAN JOURNAL OF CARDIOLOGY, vol. 85, no. 1, 2000, pages 53-57, XP001073750
- PLATZ E.A.: "epidemiologic musing on statin drugs in the prevention of advanced prostate cance" CANCER EPIDEMIOLOGY BIOMARKERS & PREVENTION, vol. 16, 2 November 2007 (2007-11-02), pages 2175-2180,
- WEI-JIAN PAN: "Lack of a clinical significant pharmacokinetic interaction between fenobibrate and pravastatin in healthy volunteers." J. CLIN. PHARMACOL., vol. 40, 2000, pages 316-323,

## Description

Oral pharmaceutical composition containing, in the same pharmaceutical form, effective amounts of pravastatin and of a PPARα, especially fenofibrate. Also described is the use of some inactive ingredients which allow to improve the dissolution and/or bioavailability of the drugs from the said composition.

### BACKGROUND OF THE INVENTION

Hypercholesterolaemia plays a crucial role in the development of atherosclerosis diseases in general and coronary heart disease in particular. The risk of progression of the atherosclerosis process to coronary heart diseases increases progressively with increasing levels of total serum cholesterol or low density lipoproteins (LDL) cholesterol at both the individual and the population level.

The HMG-CoA reductase inhibitors are reversible inhibitors of the microsomal enzyme HMG-CoA reductase, which converts HMG-CoA to mevalonate. This is an early rate-limiting step in cholesterol biosynthesis. Inhibition of HMG-CoA reductase by HMG-CoA reductase inhibitors decreases intracellular cholesterol biosynthesis, which then leads to transcriptionnally upregulated production of microsomal HMG-CoA reductase at cell surface LDL receptors.

Subsequently, additional cholesterol is provided to the cell by de novo synthesis and by receptor-mediated uptake of LDL-cholesterol from the blood. This resets intracellular cholesterol homeostasis in extrahepatic tissues, but has little effect on the overall cholesterol balance (Clin. Pharmacokinet. 1997, May, 32(5), 403-425).

The main HMG-CoA reductase inhibitors currently used in therapeutics are: pravastatin, simvastatin, lovastatin, fluvastatin, atorvastatin and cerivastatin. simvastatin, lovastatin and pravastatin are derived from fungi (14,15).
simvastatin reductase inhibitor is a clinically modified 2,2-dimethyl-butyrate analogue of lovastatin.

Fibrates are old hypolipidemic drugs with pleitropic effects on lipid metabolism. Their intimate molecular mechanisms of action have been mysterious for a long time. Recently, it has been shown that the pharmacological effect of fibrates depends on their binding to "Peroxisome Proliferator Activated Receptor alpha" (PPAR alpha). The binding of fibrates to PPAR induces the activation of the inhibition of multiple genes involved in lipid metabolism through the binding of the activated PPAR alpha to "Peroxisome Proliferator Response Element" (PPRE) located in the gene promoters. Furthermore, it was recently demonstrated that fibrates are potent antiinflammatory molecules through an indirect modulation of the nuclear-factor-Kappa B activity.

Fenofibrate or P-(4-chlorobenzoyl)-phenoxy isobutyrate isopropyl ester is useful for the treatment of adult patients with very high elevations of serum triglyceride levels and/or cholesterol levels. The usual daily dosage is 100 to 300 mg which is administered in two or three doses. Fenofibrate is absorbed as fenofibric acid which is responsible for the pharmacological activity. Fenofibric acid resulting from the hydrolysis of fenofibrate is extensively bound to plasma albumin. The plasma half-life is about 20 hours. Fenofibric acid is excreted predominantly in the urine, mainly as the glucuronide conjugate, but also as a reduced form of fenofibric acid and its glucuronides.

It has been demonstrated that the combination of a HMG-CoA reductase inhibitor and fenofibrate (administered in two separate dosage forms) was better tolerated and as efficient as a higher dose of the HMG-CoA reductase inhibitor derivative. The main disadvantage of this double administration is that it complicates the posology for the patients and hence it increases the risk of mistakes or omissions in the intake of drugs. The patient's compliance is then decreased.

Consequently, there is still a need for patients suffering from hypercholesterolemia and/or lipidemia to dispose of a pharmaceutical dosage form containing effective amounts of at least one HMG-CoA reductase inhibitor derivative and of fenofibrate and allowing to obtain a good bioavailability of both drugs.

Some patents describing association of hypolipidemiant agents are already described. For instance, US patent 6,180,660 describes methods for preventing or reducing the risk of a first occurrence of a cardiovascular event using an HMG-CoA reductase inhibitor alone or in combination with another lipid altering agent. Subjects to be treated are those having an average serum total cholesterol level, an average to mildly elevated serum low-density lipoprotein cholesterol level, and a below average serum high-density lipoprotein cholesterol level, with no history of clinically evident coronary disease.

The US Patent 6,264,938 relates to methods for treating hypercholesterolemia and atherosclerosis, and reducing serum cholesterol in a mammal. The methods of the invention comprise administering to a mammal a first amount of a bile acid sequestrant compound which is an unsubstituted polydiallylamine polymer and a second amount of an HMG CoA reductase inhibitor compound. The first and second amounts together comprise a therapeutically effective amount. The invention further relates to pharmaceutical compositions useful for the treatment of hypercholesterolemia and atherosclerosis, and for reducing cholesterol.

Pharmaceutical compositions of a fibric acid derivative associated with a HMG-CoA reductase are known and described in, for example, the European patent applications Nos. EP0455042 and EP0475148, and publications, R.L.B. Ellen, R. McPherson, American Journal of Cardiology, vol. 81, No. 4A, 1998, pages 60B-65B and M. Farnier, S. Dejager, American Journal of Cardiology, vol. 85, No. 1, 2000, pages 53-57.

The WO 01/37831 describes a pharmaceutical combination comprising separate dosage form in a common blister card of an inhibitor of the HMG-CoA reductase and a fibric acid derivative useful in the treatment at different ways of dyslipidemia of diabetics and non-diabetics.

A publication, Pan W.-J. et al, J. Clin. Pharmacol. 2000, 40, 316-323, describes a composition comprising pravastatin and fenofibrate, and the pharmacokinetical study in which bioavailability of pravastatin was compared between pravastatin alone form and the one associated with fenofibrate.

The International application No.WO 00/37078 describes a pharmaceutical composition comprising a combination of fenofibrate and cerivastatin. The application also teaches to add to the composition a glycerol derivative as solubiliser or a glyceride as liquid carrier.

US Patent 5,545,628 describes an advantageous oral pharmaceutical composition containing fenofibrate and polyglycolized glycerides while the patent PCT/BE 01/00098 describes an advantageous semi-solid oral pharmaceutical composition containing an HMG-CoA reductase.

Not described is an oral semi-solid pharmaceutical composition containing, in the same pharmaceutical form, a combination of an effective amount of pravastatin together with an effective amount of a PPARα agent, especially fenofibrate.

### SUMMARY OF THE INVENTION

The present invention relates to an oral pharmaceutical composition, containing a combination of effective amounts of pravastatin as a HMG-CoA reductase inhibitor derivative and of PPARα agent, especially fenofibrate, in the same dosage form, allowing to obtain a high bioavailability of all drugs. The invention also relates to a process for manufacturing the same.

### DETAILED DESCRIPTION OF THE INVENTION

It is an object of the present invention to disclose a pharmaceutical dosage form containing PPARα agent, especially fenofibrate, pravastatin as a HMG-CoA reductase inhibitor of the statin family, and at least one polyglycolized glyceride having a HLB balance above 10. It is another object of the present invention to disclose a pharmaceutical dosage form containing PPARα agent, especially fenofibrate, and pravastatin and at least one polyglycolized glyceride having a HLB balance above to contained in a capsule or a tablet.

Another object of the present invention is to disclose a pharmaceutical dosage form containing PPARα agent, especially fenofibrate, and pravastatin and at least one polyglycolized glyceride having a HLB balance above to with increased bioavailability for both the PPARα agent, especially fenofibrate, and pravastatin.

Also an object of the present invention is to disclose a pharmaceutical dosage form containing PPARα agent, especially fenofibrate, and pravastatin and at least one polyglycolized glyceride having a HLB balance above to from which at least the PPARα agent, especially fenofibrate, has an increased bioavailability.

Also an object of the present invention is to disclose a pharmaceutical dosage form containing PPARα agent, especially fenofibrate, and pravastatin and at least one polyglycolized glyceride having a HLB balance above to from which pravastatin has an increased bioavailability.

The formulation contains at least one polyglycolized glyceride (HLB > 10). The formulation advantageously contains one or more stabilizing agent(s) e.g. one or more antioxidant or preservative agent or a combination of both preservative and antioxidant agents.

According to the invention, a pharmaceutical composition useful for administration to a mammal comprises in a same dosage form an effective amount of at least one peroxisome proliferator activated agent (PPARα), an effective amount of pravastatin, and at least one polyglycolized glyceride having a HLB balance above 10.

Preferably, the composition comprises at least one peroxisome proliferator activated agent (PPARα) under the form of semi-solid composition (containing at least one polyglycolized glyceride having a HLB balance above 10) and pravastatin under the form of a coated tablet, both the semi-solid form and the tablet being filled in one single pharmaceutically acceptable capsule

Preferably, the composition further comprises at least one hydrophilic disintegrating agent. Examples of disintegrating agents are sodium starch glycolate, sodium croscarmellose, crospovidone, starch, colloidal silicone dioxide or another pharmaceutically accepted disintegrating agent and combinations thereof, sodium starch glycolate being preferred.

In the composition of the invention, the PPARα agent is advantageously a compound of the fibrate family, preferably a compound selected from the group consisting of fenofibrate, ciprofibrate, clofibrate, gemfibrozil, bezafibrate and combinations thereof. Especially, the PPARα agent is fenofibrate.

The pharmaceutical composition of the invention is preferably in a form suitable for the oral administration of the active agents.

The polyglycolised glyceride has an HLB balance above 10, preferably above 11, most preferably above 12.

According to an embodiment, the melting point of the said composition is below 90°C, preferably below 80°C, most preferably below 70°C.

The pharmaceutical composition of the invention contains advantageously one or more antioxidant and/or preservative agent(s), such as a vitamin E derivative and/or a methoxyphenol derivative and/or a combination thereof. The pharmaceutical composition of invention contains advantageously a wetting agent.

The composition of the invention further contains advantageously a polyethylene glycol or a mix of polyethylene glycol with different molecular mass; and/or a suspension stabilizer, such as a cellulose derivative, hydropropylcellulose.

The composition of the invention is adapted for the administration of specific amount of active agent per dose. Advantageously, the amount of PPARα, preferably fenofibrate, per dose is between 30 and 400 mg, while the amount of statin per dose is between 5 and 100mg, the amount of statin per dose being preferably lower than the amount of PPARα per dose, most preferably comprised between 0.01 and 0.5 times the amount of PPARα per dose.

The composition of the invention is for example filled in hard gelatine capsules, hypromellose capsules or in other pharmaceutically acceptable capsules.

According to a preferred detail of the composition, the composition is with the proviso that the PPARα, preferably fenofibrate, is not co-micronized, and/or, preferably and, with the proviso that the statin is not co-micronized. According to preferred embodiments, the weight ratio PPAR agent + statin/hydrophilic disintegrating agent is comprised between 100 and 0.1, advantageously between 50 and 2, preferably between 40 and 4, more preferably between 6 and 25, such as 8, 10, 12, 14, etc.

According to a further detail of a preferred embodiment, the weight ratio PPAR + statin agent/polyglycolized glyceride(s) is comprised between 10 and 0.1, advantageously between 5 and 0.2, preferably lower than 1, more preferably between 0.8 and 0.3, such as about 0.8, 0.7, 0.6, 0.5, 0.4.

The pharmaceutical composition of the invention comprises pravastatin. The composition may further contain, at least another HMG-CoA reductase inhibitor derivative of the statin family selected from the group consisting of simvastatin, lovastatin, fluvastatin, atorvastatin and cerivastatin. simvastatin, lovastatin and mixtures thereof.

The pharmaceutical composition of the invention advantageously further contains a polyethyleneglycol derivative (PEG). The amount of PEG is advantageously comprised between 0.2 and 5 times the amount of stain present in the composition, preferably between 0.5 and 2 times the amount of statin present in the composition.

According to a specific embodiment, the composition contains one or more antioxidant and/or preservative agent(s), one polyethylene derivative, and one hydrophilic wetting agent.

The semi-solid composition may be a suspension, an emulsion or a microemulsion. Pravastatin and the fibric acid derivative may be partially or totally dissolved in the semi-solid matrix formed by the excipients.

The advantages of the semi-solid formulations are multiple for pravastatin: protection of the active ingredient from air and humidity, possibility of increasing the dissolution rate of the molecule and hence of bioavailability, diminution of the risk of contamination of the operator, diminution of the risk of cross contamination, no possibility of demixing under the effect of vibrational mixing during manufacturing process, facility of the production process. The choice of the nature of the formulation of course influenced the stability of the pharmaceutical form and the bioavailability of the drug contained in it. Generally, a maximum bioavailability is achieved by preparing and keeping the drug in the amorphous/solubilized state in a solid dispersion or in a lipid-based formulation. For these systems, the barrier we are avoiding is the compound « washing-out » of solution to a large extent into a insoluble crystalline form during the dissolution/release step in vivo. These systems may consist of suspension, emulsion, microemulsion, self-emulsifying drug delivery systems (SEDDS) or self-emulsifying microemulsion drug delivery system (SMEDDS).

Microemulsions have the added advantage over suspensions such as emulsions and dispersions since thermodynamically they are more stable, that they can be manufactured with little energy input and have generally a longer shelf-life.

The formation of oil-in-water (O/W) and water-in-oil (W/O) microemulsions usually involves a combination of 3-5 basic compounds i.e. oil, surfactant, cosurfactant, water and electrolytes. The challenge is to select for a particular application oil(s) and surfactant(s) that are acceptable from a toxicological perspective and that allow to obtain a high bioavailability of the drug.

The assessment of the quality of semi-solid lipid based formulations is quite difficult since the in vitro dissolution test is of little help. Indeed, the in vitro/in vivo correlation between dissolution and bioavailability is very poor for this kind of formulation. Other analytical tools are available to the formulator to try to predict the in vivo bioavailability of isotretinoin from various formulations like the CACO-2 cells model, the assessment of the percentage of drug dissolved in the formulation, differential scanning calorimetry, microscopy,...

Nevertheless, none of them present a guarantee of in vitro / in vivo correlation and ultimately only pharmacokinetic studies on human subjects are reliable to assess the bioavailabiltiy of the drug.

Advantageously, the melting point of the final composition will be below 80°C, preferably below 60°C.

Advantageously, an emulsifier may be added (e.g distilled monoglycerides, Myverol^{®} , Gillco, US) to the formulation in order to increase the solubilization of the HMG-CoA reductase inhibitor.

Advantageously, the oral pharmaceutical composition may contain a solubilizing agent. This solubilizing agent is advantageously water and HCI soluble. An example of this kind. of solubilizing is diethylene glycol monoethyl ether (Transcutol®, Gattefossé).

Also advantageous for the stability and the bioavailability of the composition is the addition of an antioxidant agent such as either a Tocopherol derivative like Tocopherol (Vitamine E), Tocopherol acetate, Vitamine E TPGS or a methylphenol derivative like butylhydroxyanisol (BHA) or butylhydroxytoluene (BHT).

The addition of a polymer able to control the recristallisation of the active ingredient may also be useful when the active ingredient is not completely dissolved in the semi-solid matrix.

The role of the polymer is (i) to stabilize the semi-solid formulation by increasing the viscosity of the composition and (ii) to avoid the growth of particles of active ingredient that are not solubilized (or formed during the cooling of the composition) by forming a matrix in the semi- solid composition.

Examples of such agents are cellulose derivatives such as hydroxypropylcellulose, hypromellose and methylcellulose.

A wetting agent may also be added advantageously to the said composition when a very fast release in needed. Example of such agents are Na croscarmellose, Na carboxymethylcellulose or reticulated povidone. The effect of the wetting agent is strongly dependent on the nature of the active ingredient and on the nature of the semi-solid matrix.

Process for manufacturing the said pharmaceutical composition.

One of the advantages of the invention relates to the easiness of the manufacturing process of the medication and the rapidity and easiness of the pharmaceutical composition.

Briefly, the inactive ingredients are used as molten together. In an adequate tank the active ingredient is added to the molten mass and once the solution mass is homogenous, the molten is filled into pharmaceutically acceptable capsules e.g. hard gelatin capsules or hypromellose capsules. The capsules are then cooled and thereafter adequately packaged.

### Examples of formulations

### Example 1

| **Ingredient** | **mg/capsule** |
|---|---|
| ***semi-solid formulation*** | |
| Fenofibrate | 160 |
| hydropropylcellullose | 95 |
| Gelucire 44/14^{®} | 350 |
| PEG 6000 | 60 |
| | |
| ***uncoated tablet*** | |
| Pravastatin | 12,5 |
| Mannitol | 70 |
| Lactose | 15 |
| Microcrystalline cellulose | 11,5 |
| Sodium starch glycolate | 9 |
| butylhydroxyanisole | 0.01 |
| Magnesium Stearate | 2 |
| **Coating of the tablet** | |
| Absolute ethanol | 36,46 |
| Povidone | 7,29 |
| Talc | 3,64 |
| Triacetin | 0,607 |
| pro capsula una | |

The example 1 hereinabove describes a composition corresponding to the present invention wherein the fenofibrate is formulated as a semi-solid formulation and the pravastatin as a coated tablet formulation, the fenofibrate and pravastatin formulations being filled as a single composition in one single hard gelatin capsule.

### Example 2

| Ingredient | mg/capsule | | |
|---|---|---|---|
| | F7 | F8 | F9 |
| pravastatin | 30 | 40 | 25 |
| Fenofibrate | 160 | 200 | 160 |
| Gelucire 44/14^{®} | 350 | 300 | 300 |
| PEG 6000 | 20 | 20 | 30 |
| Vit E TPGS | 30 | 20 | 30 |
| Sodium starch glycolate | 20 | 10 | 20 |
| Lactose | -- | 10 | -- |
| Butylhydroxyanisole | 0.08 | 0.08 | 0.08 |
| Hydroxypropylcellulose | -- | -- | 100 |

In the present specification, the term "improved bioavailability" relates to the human bioavailability of the drug(s) in humans. The bioavailability of a drug is defined as the rate and extent to which the active substance or active moiety is absorbed from a pharmaceutical form and becomes active at the site of action. The bioavailability is essentially quantified by the area under the plasma concentration curve (AUC) and the maximal plasma concentration (Cₘₐₓ). Consequently, an improved form of the invention presents a higher bioavailability (AUC and/or Cₘₐₓ), preferably a significantly higher bioavailability than the reference, namely the actually commercialized fenofibrate form or/and the actually commercialized pravastatin form, the drug being taken via the oral route at the same dose. The preferred form of the invention presents a higher bioavailability (AUC and/or Cₘₐₓ), preferably a significantly higher bioavailability than the references which are respectively the actually commercialized form of fenofibrate and the actually commercialized form of pravastatin, when the products are taken via the oral route at the same dose. The improved bioavailability is for example improved of at least 10%, advantageously of at least 15%, preferably of at least 20% with respect to the bioavailability of the reference.

The compositions of the various formulations F7 to F9 of example 2 has been repeated, except that the fenofibrate has been replaced by ciprofibrate, clofibrate, gemfibrozil, bezafibrate, a combination of bezafibrate (50%) and fenofibrate (50%).

## Claims

1. A pharmaceutical composition useful for administration to a mammal, said composition comprising an effective amount of at least one peroxisome proliferator activated agent (PPARα), and of pravastatin and at least one polyglycolized glyceride having a HLB balance above 10.

2. The pharmaceutical composition of claim 1, wherein the PPARα agent is contained in a semi-solid vehicle containing at least one polyglycolized glyceride having a HLB balance above 10 and pravastatin is formulated as a tablet, both molecule's formulations being filled into one single pharmaceutically acceptable capsule.

3. The pharmaceutical composition of claim 1, wherein the PPARα agent is contained in a semi-solid vehicle containing at least one polyglycolized glyceride having a HLB balance above 10 and pravastatin is formulated as a coated tablet, both molecule's formulations being filled into one single pharmaceutically acceptable capsule.

4. The pharmaceutical composition of claim 1, which comprises at least one hydrophilic disintegrating agent.

5. The pharmaceutical composition of claim 1, wherein the PPARα is a compound of the fibrate family, preferably a compound selected from the group consisting of fenofibrate, ciprofibrate, clofibrate, gemfibrozil, bezafibrate and combinations thereof.

6. The composition of claim 1, wherein the PPARα agent is fenofibrate.

7. The pharmaceutical composition of claim 1, wherein the said pharmaceutical composition is to be administered via the oral route.

8. The pharmaceutical composition of claim 7, wherein the polyglycolised glyceride has an HLB balance above 11, most preferably above 12.

9. The pharmaceutical composition of claim 1, wherein the melting point of the said composition is below 90°C, preferably below 80°C, most preferably below 70°C.

10. The pharmaceutical composition of claim 1 containing one or more antioxidant and/or preservative agent(s).

11. The pharmaceutical composition of claim 10, wherein the antioxidant and/or preservative agent is a vitamin E derivative.

12. The pharmaceutical composition of claim 10, wherein the antioxidant and/or preservative agent is a methoxyphenol derivative.

13. The pharmaceutical composition of claim 10, where a combination of a vitamin E derivative and a methoxyphenol derivative is used as antioxidant and/or preservative agent.

14. The pharmaceutical composition of claim 1, wherein the composition contains a wetting agent.

15. The pharmaceutical composition of claim 4, wherein the disintegrating agent is sodium starch glycolate.

16. The composition of claim 4, wherein the disintegrating agent is sodium croscarmellose, crospovidone, starch, colloidal silicone dioxide or another pharmaceutically accepted disintegrating agent.

17. The composition of claim 1 further containing a polyethylene glycol or a mix of polyethylene glycol with different molecular mass.

18. The composition of claim 1, wherein a suspension stabilizer is added in the composition.

19. The composition of claim 18, wherein the suspension stabilizer is a cellulose derivative.

20. The composition of claim 18, wherein the suspension stabilizer is hydropropylcellulose.

21. The composition of claim 1, wherein the amount of PPARα, preferably fenofibrate, per dose is between 30 and 400 mg, while the amount of pravastatin per dose is between 5 and 100mg, the amount of pravastatin per dose being preferably lower than the amount of PPARα per dose, most preferably comprised between 0.01 and 0.5 times the amount of PPARα per dose.

22. The composition of claim 1, wherein the composition is filled in hard gelatine capsules, hypromellose capsules or in other pharmaceutically acceptable capsules.

23. The composition of claim 1, wherein the PPARα, preferably fenofibrate and/or pravastain are not co-micronized.

24. The composition of claim 1, in which the weight ratio (PPARα + pravastatin)/hydrophilic disintegrating agent is comprised between 100 and 0.1, advantageously between 50 and 2, preferably between 40 and 4, more preferably between 6 and 25.

25. The composition of claim 2, in which the weight ratio (PPARα + pravastatin)/polyglycolized glyceride(s) is comprised between 10 and 0.1, advantageously between 5 and 0.2, preferably lower than 1, more preferably between 0.8 and 0.3.

26. The pharmaceutical composition of claim 1, wherein the composition further contains a polyethyleneglycol derivative.

27. The pharmaceutical composition of claim 1, wherein the composition contains one or more antioxidant and/or preservative agent(s), one polyethylene derivative, and one hydrophilic wetting agent.

## Patentansprüche

1. Pharmazeutische Rezeptur zur Verabreichung an Säugetieren, die aus einer wirksamen Menge von mindestens einem Peroxisom-Proliferator-aktivierten Rezeptor (PPARα) und aus Pravastatin und mindestens einem polyglykolisierten Glyzerid mit einerm HLB-Wert von über 10 besteht.

2. Pharmazeutische Rezeptur aus Patentanspruch 1, das einen PPARα-Wirkstoff in einem halbfesten Vehikel mit mindestens einem polyglykolisierten Glyzerid mit einem HLB-Wert größer 10 und Pravastatin beinhaltet, ist in Tablettenform gestaltet, die Rezeptur beider Moleküle ist in einer einzigen, pharmazeutisch zugelassenen Kapsel enthalten.

3. Pharmazeutische Rezeptur aus Patentanspruch 1, das einen PPARα-Wirkstoff in einem halbfesten Vehikel mit mindestens einem polyglykolisierten Glyzerid mit einem HLB-Wert größer 10 und Pravastatin beinhaltet, ist als Dragee gestaltet, die Rezeptur beider Moleküle ist in einer einzigen, pharmazeutisch zugelassenen Kapsel enthalten.

4. Pharmazeutische Rezeptur aus Patentanspruch 1, die mindestens ein hydrophiles Sprengmittel enthält.

5. Pharmazeutische Rezeptur aus Patentanspruch 1, wobei das PPARα eine Mischung der Fibrat-Familie ist, vorzugsweise eine Mischung aus der Gruppe bestehend aus Fenofibrat, Ciprofibrat, Clofibrat, Gemfibrozil, Bezafibrat und deren Kombinationen.

6. Rezeptur aus Patentanspruch 1, wobei der PPARα Wirkstoff Fenofibrat ist.

7. Pharmazeutische Rezeptur aus Patentanspruch 1, wobei die pharmazeutische Rezeptur oral zu verabreichen ist.

8. Pharmazeutische Rezeptur aus Patentanspruch 7, wobei das polyglykolisierte Glyzerid einen HLB-Wert größer 11, vorzugsweise größer 12, hat.

9. Pharmazeutische Rezeptur aus Patentanspruch 1, wobei der Schmelzpunkt unter 90°C, vorzugsweise unter 80°C, besser unter 70°C, liegt.

10. Pharmazeutische Rezeptur aus Patentanspruch 1, die ein oder mehrere Antioxidationsmittel bzw. Konservierungsmittel enthält.

11. Pharmazeutische Rezeptur aus Patentanspruch 10, wobei das Antioxidationsmittel bzw. Konservierungsmittel ein Vitamin E Derivat ist.

12. Pharmazeutische Rezeptur aus Patentanspruch 10, wobei das Antioxidationsmittel bzw. Konservierungsmittel ein Methoxyphenol Derivat ist.

13. Pharmazeutische Rezeptur aus Patentanspruch 10, die eine Kombination eines Vitamin E Derivats und eines Methoxyphenol Derivats als Antioxidationsmittel bzw. Konservierungsmittel nutzt.

14. Pharmazeutische Rezeptur aus Patentanspruch 1, wobei die Rezeptur ein Netzmittel enthält.

15. Pharmazeutische Rezeptur aus Patentanspruch 4, wobei das Sprengmittel Natriumstärkeglycolat ist.

16. Rezeptur aus Patentanspruch 4, wobei das Sprengmittel Natriumcroscarmellose, Polyvinylpyrrolidon, Stärke, kolloidales Silikondioxid oder ein anderes pharmazeutisch zugelassenes Sprengmittel ist.

17. Rezeptur aus Patentanspruch 1, die weiterhin ein Polyethylenglykol oder eine Mischung aus Polyethylenglykol mit verschiedenen molekularen Massen enthält.

18. Rezeptur aus Patentanspruch 1, wobei ein Suspensionsstabilisator der Rezeptur hinzugefügt wird.

19. Rezeptur aus Patentanspruch 18, wobei der Suspensionsstabilisator ein Zellstoffderivat ist.

20. Rezeptur aus Patentanspruch 18, wobei der enthaltene Suspensionsstabilisator Hydropropylcelllose ist.

21. Rezeptur aus Patentanspruch 1, wobei die Menge an PPARα, vorzugsweise Fenofibrat, pro Dosis zwischen 30 und 400 mg beträgt, während die Menge an Pravastatin pro Dosis zwischen 5 and 100 mg beträgt, die Menge an Pravastatin pro Dosis vorzugsweise kleiner als die Menge der PPARα pro Dosis ist und am besten zwischen 0,01 und 0,5 Mal der Menge der PPARα pro Dosis beträgt.

22. Rezeptur aus Patentanspruch 1, wobei die Rezeptur in harte Gelatinekapseln, Hydroxypropylmethylcellulosekapseln oder in andere pharmazeutisch zugelassene Kapseln gefüllt wird.

23. Rezeptur aus Patentanspruch 1, wobei das PPARα, vorzugsweise Fenofibrat bzw. Pravastatin nicht co-mikronisiert ist.

24. Rezeptur aus Patentanspruch 1, wobei das Gewichtsverhältnis des (PPARα + Pravastatin) / des hydrophilen Sprengmittels zwischen 100 und 0,1, zweckmäßigerweise zwischen 50 und 2, vorzugsweise zwischen 40 und 4, besser zwischen 6 und 25 ist.

25. Rezeptur aus Patentanspruch 2, wobei das Gewichtsverhältnis des (PPARα + Pravastatin) / des hydrophilen Sprengmittels zwischen 10 und 0,1, zweckmäßigerweise zwischen 5 und 0,2, vorzugsweise geringer als 1, besser zwischen 0,8 und 0,3 ist.

26. Pharmazeutische Rezeptur aus Patentanspruch 1, wobei die Rezeptur darüber hinaus ein Polyethylenglykol Derivat enthält.

27. Pharmazeutische Rezeptur aus Patentanspruch 1, wobei die Rezeptur ein oder mehrere Antioxidationsmittel bzw. Konservierungsmittel, ein Polyethylen Derivat und ein hydrophiles Netzmittel enthält.

## Revendications

1. Composition pharmaceutique utile pour une administration à un mammifère, ladite composition comprenant une quantité efficace d'au moins un agent activé proliférateur de peroxysome (PPARα), et de pravastatine et au moins un glycéride polyglycolisé ayant un rapport hydrophile-lipophile supérieur à 10.

2. Composition pharmaceutique selon la revendication 1, dans laquelle l'agent PPARα est contenu dans un véhicule semi-solide contenant au moins un glycéride polyglycolisé ayant un rapport hydrophile-lipophile supérieur à 10 et la pravastatine est formulée en tant que comprimé, les formulations de l'une et l'autre molécules étant remplies dans une seule gélule acceptable sur le plan pharmaceutique.

3. Composition pharmaceutique selon la revendication 1, dans laquelle l'agent PPARα est contenu dans un véhicule semi-solide contenant au moins un glycéride polyglycolisé ayant un rapport hydrophile-lipophile supérieur à 10 et la pravastatine est formulée en tant que comprimé enrobé, les formulations de l'une et l'autre molécules étant remplies dans une seule gélule acceptable sur le plan pharmaceutique.

4. Composition pharmaceutique selon la revendication 1, qui comprend au moins un agent désintégrant hydrophile.

5. Composition pharmaceutique selon la revendication 1, dans laquelle le PPARα est un composé de la famille des fibrates, de préférence un composé choisi dans le groupe constitué du fénofibrate, du ciprofibrate, du clofibrate, du gemfibrozil, du bézafibrate et de leurs combinaisons.

6. Composition selon la revendication 1, dans laquelle l'agent PPARα est le fénofibrate.

7. Composition pharmaceutique selon la revendication 1, où ladite composition pharmaceutique est destinée à être administrée par voie orale.

8. Composition pharmaceutique selon la revendication 7, dans laquelle le glycéride polyglycolisé a un rapport hydrophile-lipophile supérieur à 11, de manière préférée entre toutes supérieur à 12.

9. Composition pharmaceutique selon la revendication 1, dans laquelle le point de fusion de ladite composition est inférieur à 90 °C, de préférence inférieur à 80 °C, de manière préférée entre toutes inférieur à 70 °C.

10. Composition pharmaceutique selon la revendication 1, contenant un ou plusieurs agents antioxydants et/ou conservateurs.

11. Composition pharmaceutique selon la revendication 10, dans laquelle l'agent antioxydant et/ou conservateur est un dérivé de vitamine.

12. Composition pharmaceutique selon la revendication 10, dans laquelle l'agent antioxydant et/ou conservateur est un dérivé de méthoxyphénol.

13. Composition pharmaceutique selon la revendication 10, où une combinaison d'un dérivé de vitamine E et d'un dérivé de méthoxyphénol est utilisée en tant qu'agent antioxydant et/ou conservateur.

14. Composition pharmaceutique selon la revendication 1, où la composition contient un agent mouillant.

15. Composition pharmaceutique selon la revendication 4, dans laquelle l'agent désintégrant est du glycolate d'amidon sodique.

16. Composition selon la revendication 4, dans laquelle l'agent désintégrant est de la croscarmellose sodique, de la crospovidone, de l'amidon, du dioxyde de silicium colloïdal ou un autre agent désintégrant accepté sur le plan pharmaceutique.

17. Composition selon la revendication 1, contenant en outre un polyéthylène glycol ou un mélange de polyéthylène glycols avec une masse moléculaire différente.

18. Composition selon la revendication 1, dans laquelle un agent stabilisant de suspension est ajouté dans la composition.

19. Composition selon la revendication 18, dans laquelle l'agent stabilisant de suspension est un dérivé de cellulose.

20. Composition selon la revendication 18, dans laquelle l'agent stabilisant de suspension est l'hydropropylcellulose.

21. Composition selon la revendication 1, dans laquelle la quantité de PPARα, de préférence de fénofibrate, par dose est comprise entre 30 et 400 mg, tandis que la quantité de pravastatine par dose est comprise entre 5 et 100 mg, la quantité de pravastatine par dose étant de préférence plus basse que la quantité de PPARα par dose, de manière préférée entre toutes comprise entre 0,01 et 0,5 fois la quantité de PPARα par dose.

22. Composition selon la revendication 1, où la composition est remplie dans des gélules en gélatine dure, des gélules d'hypromellose ou dans d'autres gélules acceptables sur le plan pharmaceutique.

23. Composition selon la revendication 1, dans laquelle le PPARα, de préférence du fénofibrate et/ou de la pravastatine ne sont pas co-micronisés.

24. Composition selon la revendication 1, dans laquelle le rapport pondéral (PPARα + pravastatine)/agent désintégrant hydrophile est compris entre 100 et 0,1, avantageusement entre 50 et 2, de préférence entre 40 et 4, plus préférablement entre 6 et 25.

25. Composition selon la revendication 2, dans laquelle le rapport pondéral (PPARα + pravastatine)/glycéride(s) polyglycolisé(s) est compris entre 10 et 0,1, avantageusement entre 5 et 0,2, de préférence inférieur à 1, plus préférablement entre 0,8 et 0,3.

26. Composition pharmaceutique selon la revendication 1, où la composition contient en outre un dérivé de polyéthylène glycol.

27. Composition pharmaceutique selon la revendication 1, où la composition contient un ou plusieurs agent(s) antioxydant(s) et/ou conservateur(s), un dérivé de polyéthylène, et un agent mouillant hydrophile.
